# EUROPEAN PATENT APPLICATION

(11) **EP 3 398 926 A1**
(43) Date of publication of application: **07.11.2018**
(21) Application number: 17169716.2
(22) Date of filing: 05.05.2017
(51) Int. Cl.: C07C 29/48, C07C 31/04, C01B 3/40

(54) **SELECTIVE ANAEROBIC OXIDATION OF METHANE ENABLES DIRECT SYNTHESIS OF METHANOL**

(71) Applicant: PAUL SCHERRER INSTITUT, 5232 Villigen PSI (CH)
(72) Inventor: SUSHKEVICH, Vitaly, 5200 Brugg (CH); PALAGIN, Dennis, 5200 Brugg (CH); RANOCCHIARI, Marco, 4310 Rheinfelden (CH); VAN BOKHOVEN, Jeroen A., 8044 Zürich (CH)
(74) Representative: Fischer, Michael

(57) **Abstract**

The present invention discloses a method for the conversion reaction of methane and water to methanol and hydrogen, comprising the steps of:
a) providing a first gas mixture comprising methane;
b) providing a promoter of the conversion reaction, said promoter comprising one or more transition metals M and/or an oxide support ZO where:
M = one or more transition metals, preferably first row transition metals, preferably copper; and ZO = alumina, silica, zeolite, titania, ceria and/or a combination thereof, preferably a zeolite;

c) applying a two-stage process wherein in the first stage the first gas mixture is brought into contact with the promoter and in the second stage the promoter is brought into contact with a second gas mixture comprising water.

Direct functionalization of methane in natural gas remains a key challenge. The invention presents in a preferred embodiment a direct stepwise method for converting methane into methanol with high selectivity (∼97%) over a copper-containing zeolite, based on partial oxidation with water. The activation in helium at 673 Kelvin, followed by consecutive catalyst exposures to 7 bars of methane and then water at 473 K, consistently produced 0.204 mole of CH₃OH per mole of copper in zeolite. Isotopic labeling confirmed water as the source of oxygen to regenerate the promoters (zeolite) active centers and renders methanol desorption energetically favorable. Based on *in situ* x-ray absorption spectroscopy, infrared spectroscopy, and density functional theory calculations, a preferred embodiment of the present invention proposes a mechanism involving methane oxidation at Cu^{II} oxide active centers, followed by Cu^{I} reoxidation by water with concurrent formation of hydrogen. Water acts as an oxidant for a direct selective partial oxidation of methane into methanol and hydrogen over a copper-exchanged zeolite.

## Description

The present invention relates to a method for the conversion of methane into methanol with high selectivity.

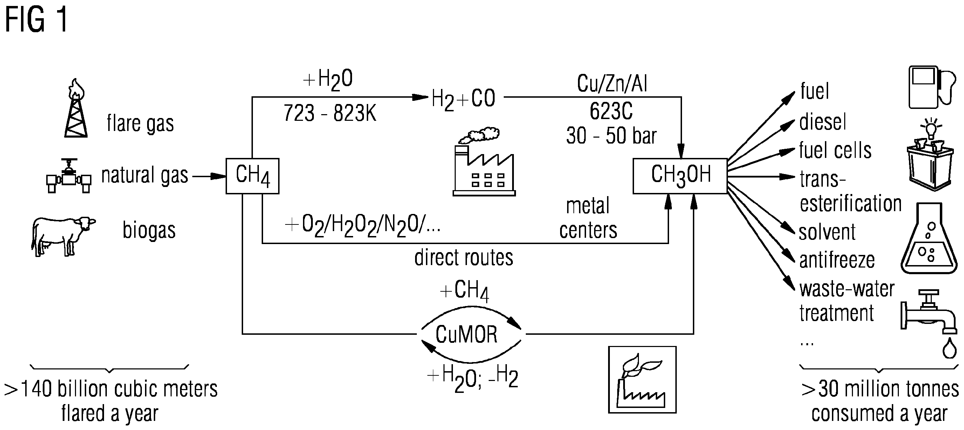

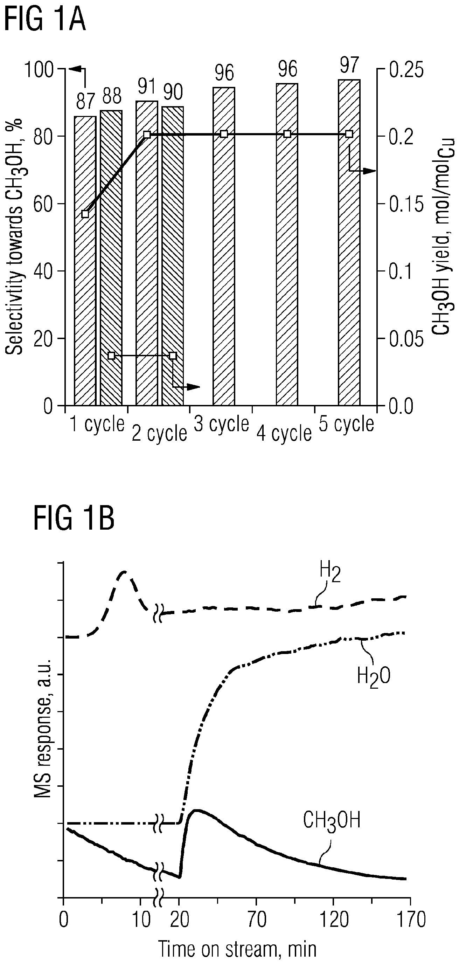

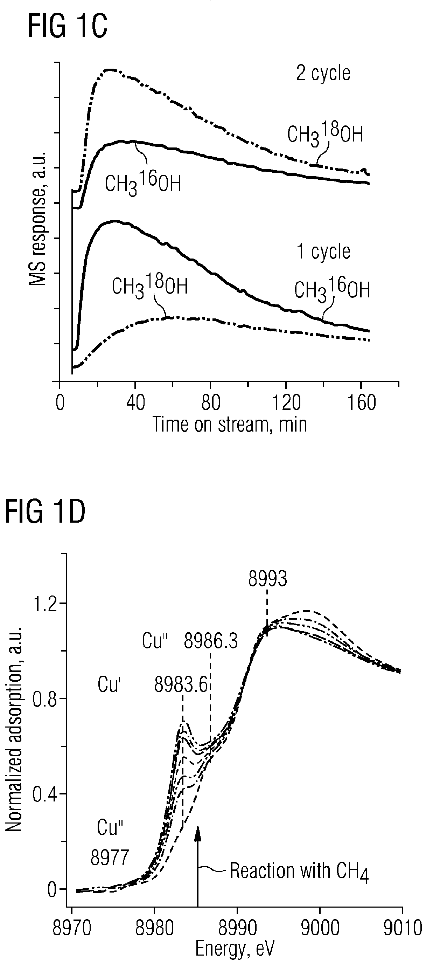

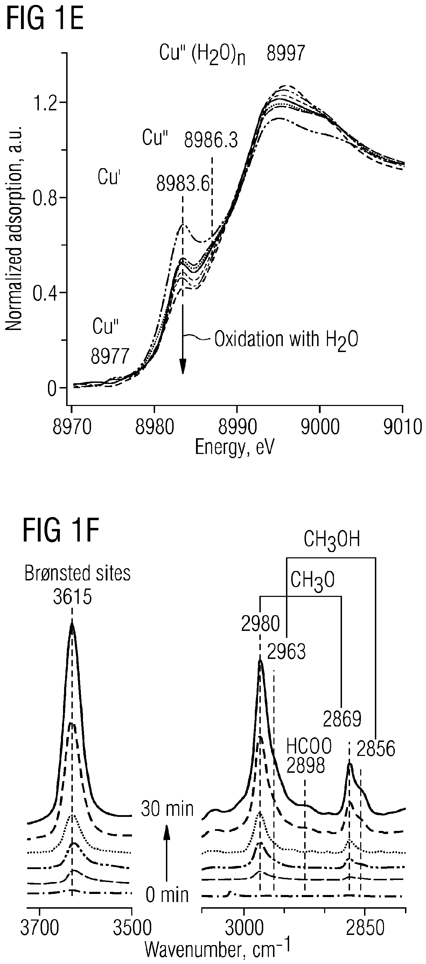

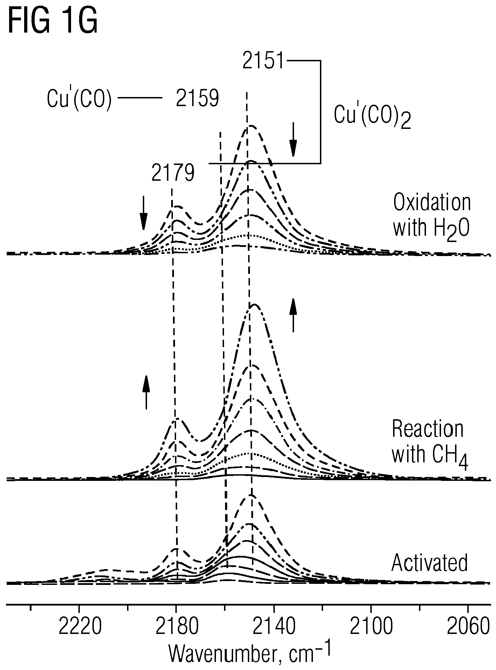

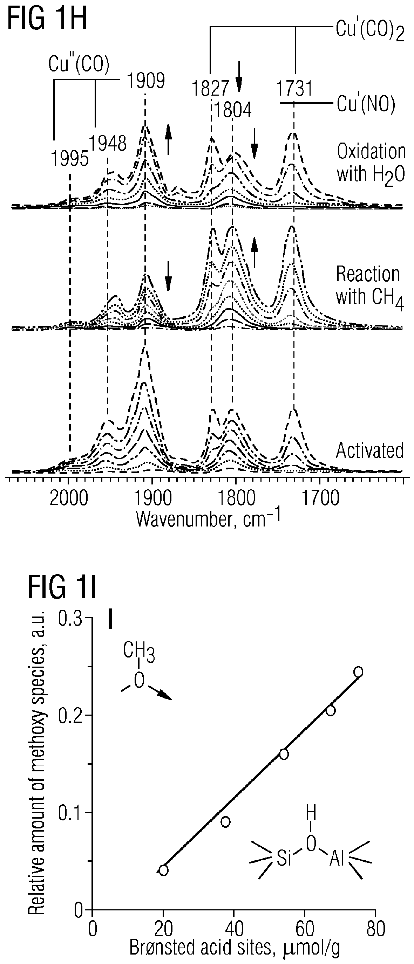

The high availability of methane as the principal component of natural gas, as well as its surge as a potent greenhouse gas, calls for the development of efficient methods for its conversion into commodity chemicals and liquid fuels. Specifically, direct conversion of methane into liquid chemicals suitable for transportation and storage still remains a major challenge. The current industrial route is indirect and relies on preliminary high temperature and high pressure oxidation to synthesis gas, a mixture of hydrogen and carbon monoxide, which can in turn react to form liquid methanol and hydrocarbons. This process, however, is only economically feasible at very large scale, and is not suitable for local, on-site applications, as, for instance, would be required to prevent flaring of the off-gas at remote oil extraction facilities. Direct routes that have been explored (Fig. 1) range from homogeneous solution-phase processes to heterogeneous chemical looping.

However, no such system has found industrial application. A promising method that has received increasing attention is the stepwise conversion of methane to methanol over metal-containing zeolites employing oxo-copper and -iron species as active sites, inspired by the natural biocatalytic enzyme systems.

Nonetheless, neither synthetic catalysts nor chemical looping systems can yet convert methane directly into methanol at high yield and productivity. For instance, there is a need to find alternative routes that circumvent the difficulty of using a strong oxidant as a source of oxygen associated with the low methanol yield or selectivity and costliness of oxidants.

Although the route *via* synthesis gas requires harsh conditions and involves two separate steps, it is used to produce nearly 100% of methanol worldwide. Direct routes using oxygen, hydrogen peroxide, or other oxidants show promise with high selectivity and/or efficiency towards methanol. Nevertheless, it is still the objective of the present invention to provide a method for the conversion of methane into methanol that does not use harsh oxidants and/or precious metal catalysts.

This objective is achieved according to the present invention by a method for the conversion reaction of methane and water to methanol and hydrogen, comprising the steps of:
a) providing a first gas mixture comprising methane;
b) providing a promoter of the conversion reaction, said promoter comprising one or more transition metals M and/or an oxide support ZO where:
   M = one or more transition metals, preferably first row transition metals, preferably copper; and ZO = alumina, silica, zeolite, titania, ceria and/or a combination thereof, preferably a zeolite;
c) applying a two-stage process wherein in the first stage the first gas mixture is brought into contact with the promoter and in the second stage the promoter is brought into contact with a second gas mixture comprising water.

The approach proposed according to the present invention using water in the second stage as an oxidant exhibits very high selectivity to methanol and requires neither harsh oxidants nor precious metal catalysts for the conversion of methane. With water acting as the oxidant, no molecular oxygen is needed. This effectively anaerobic oxidation allows an efficient low-temperature activation of methane, potentially suitable for local single-reactor conversion of the off-gas, and methane in general, into one of the main precursors for chemicals synthesis.

In order to prepare the promoter for the next contact with the first gas mixture, an additional step, which preferably include heating above room temperature, under inert gas, such as He, Ar, N₂, or under vacuum may be applied to desorb the excess water from the promoter.

In order to bring the method into a position of industrial scale conversion applicability, the two-stage process can be repeatedly executed. Each time the contact of the promoter with the second gas mixture, in particular its content of water as an oxidant supply, is completed, the promoter is re-freshed for the next conversion step with the first gas mixture thereby offering the oxygen extracted from the water as oxidant for the methane.

With respect to the reaction temperature, the first stage and the second stage may be preferably performed at a temperature range from 100 °C to 850 °C, preferably 150 to 250°C.

With respect to the reaction pressure, the first stage and the second stage may be preferably performed at a pressure range from 1 mbar to 200 bar, preferably with a pressure range for methane from 50 mbar to 35 bar and/or a pressure range for water from 5 mbar to 50 mbar.

Preferred embodiments of the present invention are hereinafter described in more detail with reference to the attached drawings which depict in:
- Figure 1: schematically the current chemical processes and the chemical process according to the present invention for converting methane to methanol;
- Figure 2: schematically the experimental data on the chemical process for the conversion of methane; and
- Figure 3: schematically the mechanism of the partial oxidation of methane using water as oxidant.

In the following a non-limiting example is given for the process of the direct conversion of methane. The direct conversion of methane into methanol over the copper-exchanged mordenite zeolite (CuMOR) was studied, using water as the source of oxygen. It can be reported here that water molecules not only act as a cheap and abundant source of oxygen to partially oxidize methane and regenerate the active sites, but also facilitate desorption of the product, as well as stabilization of the reaction intermediates. The only product of the two-electron redox reaction with water is molecular hydrogen, which itself is a valuable species. With water acting as the oxidant, no molecular oxygen is needed. This effectively anaerobic oxidation allows an efficient low-temperature activation of methane, potentially suitable for local single-reactor conversion of the off-gas, and methane in general, into one of the main precursors for chemicals synthesis.

The material for direct conversion of methane into methanol was prepared by a conventional ion exchange procedure, using an aqueous copper nitrate solution and commercial sodium mordenite with a Si/Al molar ratio of 13. The as-prepared zeolite was dried and calcined in a flow of air at 773K for 4h. The conversion of methane into methanol was studied in a multiple-cycle regime. The first cycle involved treatment of CuMOR at 673K in a flow of either oxygen or helium, followed by cooling to 473K and exposure to 7 bars of methane for 30 min, the conditions that increase the methanol yield due to the higher activity of CuMOR observed at ramped methane pressure.

Thereafter, water vapor in a flow of helium was introduced into the reactor, while the temperature was gradually increased to 673K, leading to desorption of the products. After the complete desorption monitored by mass spectrometry (MS), the second reaction cycle was started by treatment of the zeolite with a flow of dry oxygen or helium.

To directly compare the use of different oxidants, molecular oxygen was tested first. After the oxygen activation and a subsequent methane reaction, infrared spectroscopy detected methanol and methoxy species as well as over-oxidation products (carbon monoxide and formate species) on the surface of the CuMOR. The selectivity towards methanol observed in multiple cycles amounted to 85% for the oxygen-activated samples. To increase the selectivity of the process, a softer oxidant was needed to avoid the formation of strong redox sites and prevent the deep oxidation of methane. Because water was found to act as a soft oxidant, its efficiency for reactivation of CuMOR was investigated. In the first cycle, a fresh catalyst was activated under helium at 673 K, followed by methane reaction at 473 K, and methanol desorption upon water admission. A low selectivity of of 87% and 0.142 mol (CH₃OH)/mol(Cu) was detected. In the second cycle, after activation in He flow at 673K, both selectivity and methanol yield increased significantly, suggesting that water is able to regenerate the active sites (Fig. 2A). Both selectivity and yield of methanol remained constant over three additional cycles; the productivity stabilized at 0.202 mol (CH₃OH)/mol(Cu) and selectivity achieved 97%.

Reoxidation of the active sites and desorption of methanol from the CuMOR surface were induced by the presence of water vapor at 473K and 1 bar. Introduction of water led to an increase of the mass spectral water signal, as well as the signal of the methanol desorbing from the zeolite (Fig. 2B). Additionally, the formation of hydrogen was observed before the appearance of methanol in the stream. This suggests the splitting of water, with the oxygen atom used to reoxidize the copper sites, and the hydrogen atoms assembled to form molecular hydrogen, which can easily desorb from the surface of the zeolite. The oxygen atoms become a part of the active center and react to a new methane molecule in the subsequent cycle (Fig. 2A).

To confirm the insertion of oxygen atoms from water into the carbon-hydrogen bond of methane, the above experiment was repeated with ¹⁸O-labeled water, and compared the isotopic composition of methanol from both the unlabeled and ¹⁸O-labeled water-reactivated zeolite by MS (Figs. 2C).

In the first cycle, mostly unlabeled methanol was formed, accompanied by the delayed release of CH₃¹⁸O, indicating minor oxygen exchange on the surface of CuMOR. In contrast, after full desorption of methanol, followed by heating in helium at 673K and a second interaction with methane, the MS signal from the labeled methanol increased significantly (Fig. 2C). Simultaneously, the signal from the unlabeled methanol decreased, pointing to the presence of a significant number of ¹⁸O atoms in the active sites of CuMOR after interaction with water.

Such a redox process should be accompanied by a change in the copper oxidation state, which was monitored by *in situ* x-ray absorption spectroscopy (XAS) and Fourier transform infrared spectroscopy (FTIR) of adsorbed probe molecules; these methods have been successfully applied previously to the study of many redox materials, including copper exchanged zeolites. The activation and reaction protocols were identical to those described for the reaction tests. The x-ray absorption near edge structure (XANES) spectrum of activated CuMOR showed a pre-edge feature at 8977 eV, characteristic of Cu^{II}, the absorption edge energy of 8993 eV, and a small shoulder at 8986.3 eV, which are indicative of a dominant copper oxidation state of Cu^{II}. A shoulder at 8983.6 eV indicates the presence of a minor fraction of Cu^{I}. The formation of Cu^{I} atoms during the activation of CuMOR in helium is due to the autoreduction process, which is commonly observed for copper-exchanged zeolites starting at 473K. Depending on the reaction conditions, along with the formation of different side-products, the autoreduction process can lead to tarnishing the catalyst activity, as it causes the transformation of Cu^{II} species into Cu^{I} that are not active in methane oxidation.

Figure 2 shows in detail the experimental data:
A) Methanol yield and selectivity across multiple cycles, each involving a helium activation at either 673K (red line and blue bars) or 473K (yellow line and green bars), followed by methane reaction and then catalyst reactivation by water at 473K;
B) Mass spectral responses for hydrogen (m/z = 2), water (m/z = 18), and methanol (m/z = 31) after the interaction with methane at 473K and 7 bars, followed by purge with water vapor in helium (2.6 vol%, 1 bar, total flow = 40 ml/min);
C) Mass spectral responses for unlabeled (m/z = 31) and ¹⁸O-labeled (m/z = 33) methanol after first and second reactivation using labeled H₂¹⁸O, respectively;
D) *in situ* XANES spectra recorded during the interaction of CuMOR (pre-treated in helium flow) with methane at 473K;
E) *in situ* XANES spectra recorded during the interaction of water vapor with CuMOR at 473K and 1 bar after the methane reaction;
F) time-resolved *in situ* FTIR spectra of surface species formed during the interaction of CuMOR (pre-treated in a flow of helium) with 7 bars of methane at 473K ;
G) FTIR spectra of CO adsorbed at 100K onto CuMOR that was vacuum-activated (bottom), reacted with methane (center) and reoxidized with water vapor (top);
H) FTIR spectra of NO adsorbed at 100K onto CuMOR that was vacuum-activated (bottom), reacted with methane (center) and reoxidized with water vapor (top); and
I) relative number of methoxy species vs. number of Brønsted acid sites formed during the interaction of methane with CuMOR at 473K within 5 to 120 min.

Upon interaction with methane, the intensity of the peak due to Cu^{I} species increased significantly, hence confirming the reduction of copper by methane leading to the formation of oxidation products (Fig. 2D). Simultaneously, the oxygen atoms of the active sites have reacted with methane, forming methanol or other oxidation products. Linear combination fitting (LCF) showed a decrease in the number of Cu^{II} species (up to 70% of Cu^{II} is converted into Cu^{I}). The interaction with methane, therefore, leads to the reduction of Cu^{II} sites into Cu^{I}. To determine the oxidation state of copper during the interaction with water, the latter was introduced into the reaction cell and time-resolved x-ray absorption spectra were acquired. The XANES data showed that as soon as the water was introduced, the intensity of the peak at 8983.6 eV decreased, indicating the oxidation of Cu^{I}. Simultaneously, the peak at 8997 eV due to the hydrated Cu^{II} species appeared in the spectrum, hence confirming that water oxidized Cu^{I} sites into Cu^{II} (30% of Cu^{I} was oxidized into Cu^{II}, Figs. 2E). After two hours, 40% of Cu^{I} sites were not fully reoxidized by water (Fig. 2E), which indicates that not all copper sites are reactive towards water. This could be due to co-existing of different types of copper active species that have been identified in CuMOR.

To differentiate between various copper sites, FTIR spectroscopy of adsorbed carbon monoxide and nitric oxide, the most common probe molecules for studying Cu^{I} and Cu^{II} species was used (Fig. 2G, H for CO and NO, respectively). Before each measurement, the CuMOR sample was pre-heated in vacuum at 673K for 2h. At low CO coverages (pressure < 0.01 torr), the exclusive formation of Cu^{I} monocarbonyl surface species was observed (2159 cm⁻1). Increasing the carbon monoxide pressure led to the appearance of new bands due to the low (2151 cm⁻¹) and high (2179 cm⁻¹) frequency vibrations of Cu^{I} (CO)₂ dicarbonyl species (Fig. 2G).

As was observed by XAS, the presence of Cu^{I} in the activated samples relates to autoreduction. After the interaction with methane, the intensity of both bands increased significantly, due to the formation of additional Cu^{I} sites. The positions of all three bands did not shift, indicating that the nature of copper sites interacting with methane and those undergoing autoreduction is similar. As expected, the subsequent interaction of the sample with water led to the partial reoxidation of Cu^{I} sites, observed by the decrease of the bands at 2150-2180 cm⁻¹.

As the FTIR of the adsorbed carbon monoxide does not allow monitoring of the transformation of Cu^{II} species, NO was used to access the changes in copper oxidation state. The data obtained after NO adsorption on the CuMOR sample activated in vacuum show the presence of bands at 1804, 1731, and 1827 cm⁻¹ that are associated with NO bound to Cu^{I} sites, as well as 1909, 1948, and 1995 cm⁻¹ assigned to different Cu^{II} species (Fig. 2H). After subsequent interaction of the freshly vacuum-activated CuMOR with methane at 473K, the spectra of adsorbed NO showed a significant increase in the concentration of Cu^{I} sites, accompanied by simultaneous decrease of the quantity of Cu^{II} sites, in line with both the FTIR spectra of the adsorbed carbon monoxide and XAS. After the vacuum-activated CuMOR sample reacted with methane, and subsequently with water, low temperature adsorption of NO revealed a decrease of the bands associated with Cu^{I} and an increase of the bands associated with Cu^{II}. In agreement with the XAS data, reoxidation with water did not induce full conversion of Cu^{I} into Cu^{II}. Thus only a fraction of copper sites that can activate the C-H bond of methane can be reoxidized with water, while the rest of sites are not active. This explains the non-stoichiometric oxidation of methane with CuMOR, leading to a lower yield of methanol compared to the theoretical maximum (0.202 mol (CH₃OH)/mol(Cu) vs. 0.5 mol (CH₃OH)/mol(Cu)).

The above results describe the processes that occur at the active sites of CuMOR without addressing the transformation of methane. To study the initial stages of methane activation over CuMOR, time-resolved *in situ* FTIR spectroscopy was applied. As discussed above, the reaction of the helium-activated CuMOR with methane led to the formation of different surface species, including molecular methanol and methoxy groups (fig. S6). At the same time, a band at 3615 cm⁻¹ appeared, which is typical of Brønsted acid sites of the H-MOR zeolite (Fig. 2F). The number of observed Brønsted acid sites estimated from the FTIR spectroscopy of adsorbed pyridine scaled linearly with the total number of methoxy surface species (Fig. 2I). This indicates that the C-H bond cleavage coincides with methoxy and Brønsted acid site formation. The capacity of water to reactivate the oxidation sites of CuMOR suggests the possibility of partial methane oxidation without the need for temperature cycling. To test this, two cycles were carried out in which the CuMOR activation in a flow of helium, the reaction with methane and the water reoxidation all occurred at 473K without high temperature treatment (Fig. 2A). *In situ* FTIR and MS confirmed the formation of methoxy species and methanol over the fresh CuMOR as well as reoxidation of the material with water in the second cycle, indicating the feasibility of isothermal conversion of methane into methanol. However, the yield of methanol obtained for 473K activation was significantly lower (0.04 mol (CH₃OH)/mol(Cu)) compared to the high temperature activated CuMOR. This is caused by the poisoning effect of water, which was not desorbed at 473K. It has been shown that a higher methane pressure has to be applied in the isothermal regime to compensate for the loss of activity. Following this protocol, one was able to partially recover the zeolite activity, which amounted to 0.08 mol (CH₃OH)/mol(Cu) at 25 bars of methane.

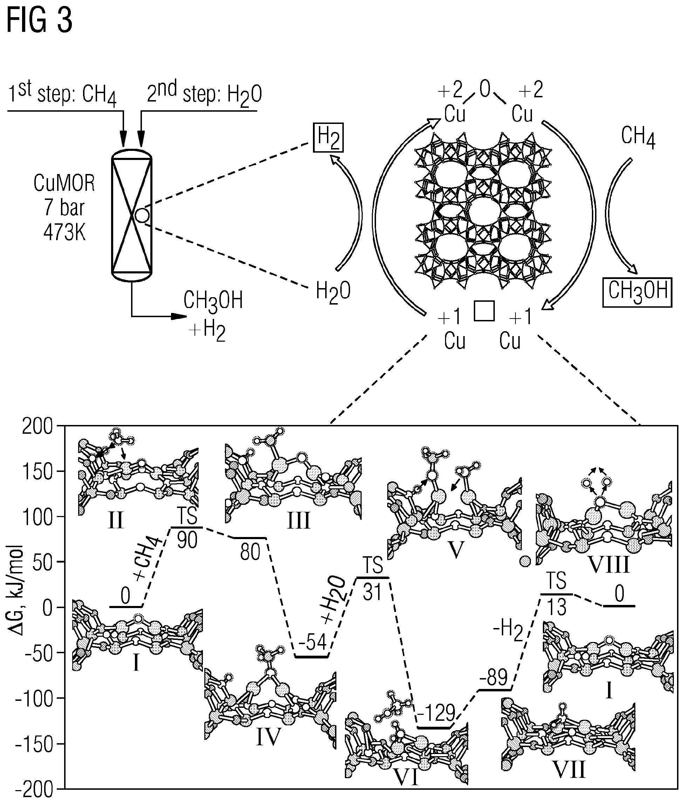

The unique behavior of CuMOR relates to the reactivity of zeolite-supported copper oxide species. Overwhelming evidence in the literature suggests that there is no unique species, which is solely responsible for zeolite activity, with multitude of copper oxide clusters, such as monomeric, dimeric, and multimeric copper species suggested as active towards methane in various zeolites. However, the limited size of the pores in mordenite stabilizes dicopper sites with bridged oxygen that mimic active sites of enzymes. Suitably, such an active center provides two electrons to stoichiometrically oxidize methane into methanol. The reactive oxygen in the Cu-O-Cu core could react with methane, yielding methoxy and reducing Cu^{II} into Cu^{I}. Instead of the common reoxidation with oxygen, it was showed that water returns two electrons, yielding reactive Cu^{II} in concert with hydrogen formation (Fig. 3, upper panel). To explain the oxidative behavior of water and to obtain detailed insight into the reaction mechanism, the feasibility of such a process was studied by density functional theory (DFT). It was initially focused on the above described mono(µ-oxo)dicopper site, which supports the two-electron process, and has the correct stoichiometry for typical ion-exchanged zeolite systems.

Fig. 3 (lower panel) shows the calculated reaction profile. Mono(µ-oxo)dicopper reacts with methane *via* the formation of a CH₃ fragment bound to a copper atom of the CuMOR active site and proton abstraction by the zeolite framework, leading to the formation of a new Brønsted site (structure III, as seen experimentally in Fig. 2F). The calculated activation barrier amounts to 90 kJ/mol (structure II), which is comparable to the previously reported values corresponding to the classical methane "rebound" mechanism. After the activation of methane on the copper atom, a methoxy species is formed (structure IV) and both copper centers are reduced to Cu^{I} (Fig. 2D, G, H). The formation of the methoxy species and the Brønsted site is exothermic by 54 kJ/mol. A subsequent direct desorption of methanol would produce a very high-energy Cu⁺□Cu⁺ fragment (see fig. S11, Structure V^{B}), which is unlikely to occur.

The upper panel schematically represents the reaction conditions of the partial oxidation of methane by water, involving the reduction of the dicopper site of mordenite, providing two electrons to stoichiometrically oxidize methane into methanol. Subsequent reduction of water into hydrogen returns two electrons for the rejuvenation of the mono(µ-oxo)dicopper active core. The lower panel shows the DFT simulated pathway, illustrating the thermodynamic and kinetic feasibility of the proposed mechanism.

The addition of water relaxes the system *via* a relatively low-energy transition state (Δ*E^{≠}* = 85 kJ/mol; structure V), facilitating reaction of the Brønsted site with the methoxy species to produce methanol and Cu^{I}-OH₂-Cu^{I}, stabilized by the formation of three additional hydrogen bonds (structure VI). Subsequent desorption of methanol (structure VII) is followed by the two-electron reduction process, leading to the formation of molecular hydrogen (structure VIII, as seen experimentally in Fig. 2B), and, thus, regeneration of the original Cu^{II}-O-Cu^{II} active site (Structure I). The activation energy of the reduction process is calculated to be 102 kJ/mol comparable to the methane activation barrier, thus making such a process thermodynamically and kinetically feasible.

The role of water is, therefore, twofold. First, it regenerates the catalyst by providing an oxygen atom for the two-electron reoxidation of the copper active center. The unique configuration of the mono(µ-oxo)dicopper active site enables water to act as an oxidant. Second, the presence of water ensures energetically favorable desorption of methanol. An excess of water molecules provides additional stabilization of the reaction intermediates and the transition states (see figs. S13 and S14), making the proposed mechanism more feasible. Further DFT calculations (see Fig. S15) on copper trimer revealed that a similar mechanism of Cu^{II} reduction by methane and subsequent reoxidation and hydrogen formation with water is equally likely for other species containing multiple copper atoms.

The anaerobic direct oxidation of methane to methanol demonstrated here is promising for cost- and energy-efficient, local, on-site applications. The use of water instead of oxygen contributes to development of an industrial process for the direct methane to methanol conversion.

## Claims

1. A method for the conversion reaction of methane and water to methanol and hydrogen, comprising the steps of:
a) providing a first gas mixture comprising methane;
b) providing a promoter of the conversion reaction, said promoter comprising one or more transition metals M and/or an oxide support ZO where:
M = one or more transition metals, preferably first row transition metals, preferably copper; and ZO = alumina, silica, zeolite, titania, ceria and/or a combination thereof, preferably a zeolite;
c) applying a two-stage process wherein in the first stage the first gas mixture is brought into contact with the promoter and in the second stage the promoter is brought into contact with a second gas mixture comprising water.

2. The method according to claim 1 wherein an additional step, which preferably include heating above room temperature, under inert gas, such as He, Ar, N₂, or under vacuum is applied to desorb the excess water from the promoter.

3. The method according to claim 1 or 2, wherein the two-stage process is repeatedly executed.

4. The method according to any of the preceding claims, wherein the first stage and the second stage are performed at a temperature range from 100 °C to 850 °C, preferably 150-250°C.

5. The method according to any of the preceding claims, wherein the first stage and the second stage are performed at a pressure range from 1 mbar to 200 bar, preferably with a pressure range for methane from 50 mbar to 35 bar and/or a pressure range for water from 5 mbar to 50 mbar.
